Europäisches Patentamt

European Patent Office

Office européen des brevets

(11) Publication number: 0 369 797
A2

## (12) EUROPEAN PATENT APPLICATION

(21) Application number: 89311898.4

(22) Date of filing: 16.11.89

(51) Int. Cl.⁵: C12P 21/08, G01N 33/577, G01N 33/68, C12N 15/23, C12N 15/62

(30) Priority: 16.11.88 JP 289591/88

(43) Date of publication of application:
23.05.90 Bulletin 90/21

(84) Designated Contracting States:
AT BE CH DE ES FR GB GR IT LI LU NL SE

(71) Applicant: SHIONOGI SEIYAKU KABUSHIKI KAISHA trading under the name of SHIONOGI & CO. LTD.
3-1-8, Dosho-machi, Chuo-ku
Osaka-shi, Osaka(JP)

(72) Inventor: Okamoto, Hiroshi
3-205 Tsunogoro-Jutaku 2-15, Tsunogoro
Sendai-Shi Miyagi-Ken(JP)
Inventor: Ide, Misao
3-22, Sanno-Cho
Nishinominya-Shi Hyogo-Ken(JP)
Inventor: Yoshida, Nobuo
5-8, Koshiensanban-Cho
Nishinomiya-Shi Hyogo-Ken(JP)
Inventor: Teraoka, Hiroshi
2-47-10, Takakuradai
Sakai-Shi Osaka(JP)

(74) Representative: Nash, David Allan et al
Haseltine Lake & Co. 28 Southampton
Buildings Chancery Lane
London WC2A 1AT(GB)

(54) A monoclonal antibody recognizing a reg protein.

(57) A monoclonal antibody recognizing a reg protein and a hybridoma producing the monoclonal antibody are provided. Also, methods for the assay of a reg protein using the monoclonal antibody are provided.

EP 0 369 797 A2

## A MONOCLONAL ANTIBODY RECOGNIZING A REG PROTEIN

The present invention relates to monoclonal antibodies recognizing regenerating protein (hereinafter referred to as reg protein), hybridomas producing the said antibodies, and methods for the assay of the reg protein.

When nicotinamide is administered to partially pancreatectomized rats, regeneration of pancreatic islets of Langerhans and a decrease in urine glucose levels is observed after approximately one month. Moreover, as this occurs, pronounced expression of a certain gene (i.e., reg gene) is noted (K. Terazono et al., J. Biol. Chem. 263: 5, 2111-2114 (1988)). The expression of this regenerating gene (reg gene) is also observed in pancreatic islets of Langerhans which manifest hyperplasia as a result of treatment with aurothioglucose. Therefore, this reg gene and the protein coded by this gene (i.e., reg protein) are presumed to play important roles in the proliferation and expression of cells in the pancreatic islets of Langerhans. Moreover, since the presence of a reg gene homologous with the aforementioned rat reg gene in cDNA libraries originating from the human pancreas has been demonstrated, it is presumed that reg genes and reg proteins should perform the same functions in the human pancreatic islet cells as in rat pancreatic islet cells.

Therefore, if monoclonal antibodies specifically recognizing reg proteins, or analytical reagents specifically binding to the reg proteins, were available, then this would provide extremely useful means for the detection, identification, assay and purification of reg proteins, as well as the elucidation of the biological action of such proteins. Moreover, the availability of such antibodies or reagents would also be expected to provide useful techniques for the diagnosis and therapy of diseases involving abnormalities in the expression of reg proteins.

According to one aspect of this invention, there is provided a monoclonal antibody which recognizes a reg protein.

In a preferred embodiment, the reg protein is of human or rat origin.

In a particularly preferred embodiment, the monoclonal antibody is selected from the group consisting of monoclonal antibody REG 5, monoclonal antibody REG 17, monoclonal antibody REG 30, monoclonal antibody HREG N1 and monoclonal antibody HREG 9.

In another aspect, this invention provides a hybridoma which produces a monoclonal antibody of the first aspect of the invention.

By analogy with the foregoing, a preferred hybridoma is one selected from the group consisting of hybridoma REG 5, hybridoma REG 17, hybridoma REG 30, hybridoma HREG N1, and hybridoma HREG 9.

In a further aspect of the invention, there is provided a method for the assay of a reg protein of this invention which comprises the steps of, preparing a first antibody that specifically recognizes a reg protein, adding an analyte containing the said reg protein so as to bind the said reg protein to the said first antibody, adding a second antibody that specifically recognizes the reg protein so as to bind the said second antibody to the said conjugated reg protein, adding a third antibody that specifically recognizes the said second antibody so as to bind the said third antibody to the said conjugated second antibody, and determining the amount of the said reg protein by measuring the amount of the said third antibody.

In a preferred embodiment, the second antibody is an IgM and the third antibody is an anti-IgM antibody.

Another method for the assay of reg protein embodying this invention comprises the steps of preparing a first antibody that specifically recognizes a reg protein, adding an analyte containing the said reg protein so as to bind the said reg protein to the said first antibody, adding a labelled second antibody that specifically recognizes the said reg protein so as to bind the said second antibody to the said conjugated reg protein, and determining the amount of the said reg protein by measuring the amount of the said labelled second antibody.

Thus, the invention described herein makes possible the objectives of: (1) providing a monoclonal antibody specifically recognizing rat or human reg protein; (2) providing a monoclonal antibody of the aforementioned type which can be utilized in the detection, identification, assay and purification of reg protein as well as the diagnosis and treatment of colorectal cancer and other diseases; and (3) providing a method of detection and assay of the reg protein employing the aforementioned monoclonal antibody.

For a better understanding of this invention and to show how the same can be carried into effect, reference will now be made, by way of example only, to the accompanying drawings wherein:

Figure 1 shows a calibration curve for the sandwich assay of reg protein using REG 5 and REG 17-peroxidase conjugate.

Figure 2 shows a calibration curve for the sandwich assay of reg protein using REG 5, REG 17 and an anti-IgM antibody.

Figure 3 shows the base sequence of rat reg gene cDNA and the amino acid sequence deduced from the said base sequence.

Figure 4 is a schematic diagram illustrating the construction of an expression plasmid for a fusion protein of human interferon- γ and rat reg protein.

Figure 5 shows the base sequence of the human reg gene cDNA and the amino acid sequence deduced from the said base sequence.

Figure 6 is a schematic diagram illustrating the construction of an expression vector for human reg protein.

Figure 7 shows a calibration curve for the sandwich assay of human reg protein using HREG N1 and HREG 9-peroxidase conjugate.

The monoclonal antibodies of the present invention are prepared by using recombinant reg protein as the immunogen.

Natural reg protein, appropriately extracted and purified, may also be used as the immunogen in the present invention. However, as mentioned above, the reg gene has already been obtained, and therefore the use of the recombinant reg protein, obtained by expression of the said gene in suitable microorganisms, is convenient and practically desirable. As regards genetic engineering methods for the manufacture of reg protein, specific procedures are detailed in Japanese Patent Application Nos. 63-71671 and 63-195727. However, the applicable methods are by no means limited to the procedures described in the said two patent applications; as indicated in Example 1, other proteins, for example, a fusion protein of human interferon-γ and reg protein, may also be employed for the present purpose. Using such reg proteins or their fusion proteins, hybridomas which produce monoclonal antibodies recognizing the target reg protein can be prepared.

The aforementioned immunogens are emulsified in Freund's complete adjuvant or other suitable adjuvant and then used for the immunization of mice. Immunization is performed by repeated inoculation of the mice with the aforementioned emulsion several times at intervals of several weeks, via the intraperitoneal, subcutaneous or intravenous route. Then, 2 to 7 days after the final immunization, the spleens are excised and used as the source of antibody-producing cells. Myeloma cell lines possessing appropriate selectable markers such as hypoxanthine guanine phosphoribosyl transferase deficiency (HGPRT⁻) or thymidine kinase deficiency (TK⁻) are prepared for use as the parent cells to be fused with these antibody-producing cells in order to obtain the desired hybridomas.

Thus, hybridomas are created by fusion of the said myeloma cells and antibody-producing spleen cells. Media suitable for use in the preparation of hybridomas are prepared appropriately by adding about 10% of fetal calf serum (FCS) to any of the media ordinarily used for cell cultures, such as Eagle's MEM, modified Dulbecco's medium or RPMI-1640, etc.

First, the parent cells, i.e., myeloma and spleen cells, are prepared. The efficiency of cell fusions can be increased by the use of a fusing agent such as polyethylene glycol at a concentration of approximately 50%. The fused strains are selected by the HAT selection method. Then, using the supernatants, the hybridomas so created are screened by any of the conventional methods such as membrane immunofluorescence, enzyme linked immunosorbent assay (ELISA), immunohistological staining or radioimmunoassay (RIA), thereby selecting hybridoma clones which secrete the desired immunoglobulins. In order to examine the monoclonicity of the hybridomas, after plating normal spleen cells, if necessary, onto the microwells as a feeder layer, the hybridoma cells are plated so that no more than a single hybridoma cell appears in each well, and the clones grown in this manner are again screened. Monoclonal hybridomas are finally obtained by repetition of this subcloning process.

Next, in order to prepare the monoclonal antibodies of the present invention, the hybridoma obtained in the manner described above is grown either in a culture vessel (i.e., in vitro) or within the body of an animal (i.e., in vivo). For growth in vitro, a culture medium is prepared by adding FCS to any of the ordinary media mentioned above, and after 3 to 5 days of cultivation in this medium, monoclonal antibodies can be obtained from the supernatant of the medium. As regards in vivo growth, the hybridoma is inoculated into the peritoneal cavity of a mammalian host, then after about one week the ascitic fluid is collected and monoclonal antibodies are obtained from the said ascites.

The monoclonal antibodies obtained from the supernatant of the culture medium or ascites in this manner can be purified by an appropriate combination of conventional processes such as ammonium sulphate fractionation, gel filtration, etc.

The monoclonal antibodies REG 5, REG 17 and REG 30 of the present invention were all obtained using a fusion protein of human interferon- γ and rat reg protein as the immunogen. As indicated in examples, these antibodies display reactivity with both mature human reg protein and with mature rat reg

3

protein. The monoclonal antibodies HREG N1 and HREG 9 of the present invention were obtained using as immunogens mature human reg protein expressed in yeast. These antibodies display reactivity with mature human reg protein. In view of the high degree of homology between human and rat reg proteins, it seems that reg protein is present in all mammals, and accordingly that monoclonal antibodies of the present invention could recognize all mammalian reg proteins. That is, the present invention provides monoclonal antibodies capable of recognizing the reg proteins of all mammalian species.

The hybridomas REG 5, REG 17 and REG 30 which produce the respective monoclonal antibodies REG 5, REG 17 and REG 30 of the present invention were designated Hybridoma REG 5, Hybridoma REG 17 and Hybridoma REG 30 and deposited with the Fermentation Research Institute Agency for Industrial Science and Technology, 1-3, Higashi 1 chome, Tsukuba-shi, Ibaraki-ken, Japan on November 8, 1988 under Accession numbers FERM BP-2134, 2135 and 2136 respectively in accordance with the Budapest Treaty. The hybridomas HREG 9 and HREG N1, which produce the respective monoclonal antibodies HREG 9 and HREG N1 of the present invention were designated Mouse Hybridoma HREG 9 and Mouse Hybridoma HREG N1 and deposited with the aforementioned Fermentation Research Institute Agency for Industrial Science and Technology on September, 1989 under the Accession numbers FERM BP-2591 and 2592 respectively in accordance with the Budapest Treaty.

The applicable methods for immunological assay of reg protein using the monoclonal antibodies of the present invention include, of course, RIA and EIA techniques employing one kind of antibody, and RIA techniques employing two kinds of antibodies, however, the sandwich EIA method is most appropriate.

The materials which can be used as the solid phase for immobilization of the antibody include any of the commercially available solid supports used for antigen-antibody reaction ordinarily employed for immunoassay, for example, glass or synthetic resin beads or balls, tubes, plates, etc. The monoclonal antibodies recognizing reg protein of the present invention are adsorbed onto these supports. Ordinarily, the antibody is adsorbed onto one of these supports by leaving the antibody and support in a sodium bicarbonate solution or phosphate buffer solution at pH 6 to 10 overnight at 4°C or for 1 hour at 37°C. The support bearing the adsorbed antibody is stored at a suitably low temperature in the presence, if necessary, of an appropriate preservative such as sodium azide.

After purification, the antibodies of the present invention can be used without further modification, but the $F(ab')_2$ or $Fab'$ fragments obtained from the antibodies can also be used. Purification can be effected by subjecting the ascites or culture medium supernate containing the antibodies to sodium sulphate or ammonium sulphate fractionation, column chromatography or gel filtration, etc. The IgG so obtained is cleaved by pepsin digestion to form the $F(ab')_2$ fragment, and if this fragment is, furthermore, reduced with 2-mercaptoethylamine, the $Fab'$ fragment is obtained. The preparation of the $Fab'$ fragment from IgG is disclosed in detail in the Journal of Immunoassay, 4, 209-327 (1983), and the same procedure can be used in connection with the present invention.

The antibody can be labelled with enzymes such as alkaline phosphatase, $\beta$-D-galactosidase, peroxidases, glucose oxidase, etc. For the purposes of the present invention, horseradish peroxidase is particularly desirable.

The antibody can be conjugated with the enzyme as follows. For example, in the case of a peroxidase, as indicated in examples described below, the hydroxyl groups in the sugar chains of the enzymes are oxidized to form aldehydes, and these can be chemically bound to the amino groups of the antibody.

The antibodies can also be labelled through cross-linking agents, for example, N-N'-o-phenylene dimaleimide, 4-(N-maleimidomethyl)cyclohexanecarboxylic acid N-succinimide ester, 6-maleimidohexanoic acid N-succinimide ester, 3-(2-pyridyldithio)propyonic acid N-succinimide ester, 4,4'-dithiodipyridine or other well-known cross-linking agents. The reactions of these cross-linking agents with the enzyme and antibody are conducted in accordance with well-known procedures, chosen in accordance with the properties of the cross-linking agent that is used.

Immunoassay systems of higher sensitivity can be established if the aforementioned enzyme-labelled antibodies are purified by affinity chromatography or the like. After addition of thimerosol or glycerol as a stabilizer, or after lyophilization, the purified enzyme-labelled antibody is stored in a cold, dark environment until required for use.

The assay of reg protein can be accomplished by using two varieties of reg protein-recognizing monoclonal antibodies, and then effecting a reaction with an antibody which specifically recognizes one of the previous two antibodies.

For example, the monoclonal antibody REG 5 (classified as IgG) is immobilized, and to this is added an analyte containing reg protein, thereby binding the said reg protein to the antibody. Next, the monoclonal antibody REG 17 (classified as IgM) is allowed to react and is thereby bound to the reg protein. Next, a third antibody specifically recognizing IgM is bound to this complex. Then, the amount of reg protein is determined by measuring the amount of bound third antibody. The aforementioned third antibody can readily be detected, for example, by prior or subsequent labelling with an enzyme or other suitable label.

In addition to the aforementioned method, the conventional sandwich assay technique is also applicable for the present purpose. In order to measure reg protein by this technique, first, a monoclonal antibody specific for reg protein is immobilized. To this is added the analyte containing reg protein, thereby binding the said reg protein to the antibody. Next, a second monoclonal antibody labelled with an enzyme or other suitable label is allowed to react and thereby bound to the reg protein. Thus, the reg protein can be determined by measuring the amount of the label of the aforementioned second monoclonal antibody.

In the following, the present invention will be described in detail by means of specific examples, however, these examples are merely illustrative and by no means limit the scope of the present invention.

Example 1

Preparation of immunogen

Expression of mature rat reg protein fused with human interferon- γ

(a) Construction of expression vectors

All enzymes used were purchased from the Takara Shuzo Co., Ltd.

The Escherichia coli strain used for expression was C600 (F⁻, thi-1, thr-1, leuB6, lacY1, tonA21, supE44, λ⁻). The recombinant DNA techniques were performed in accordance with the procedures indicated in "Molecular Cloning" (Cold Spring Harbor Laboratory, 1982). DNA fragments were isolated by elution from agarose gel (Nucl. Acids Res. 8, 253-264 (1980)).

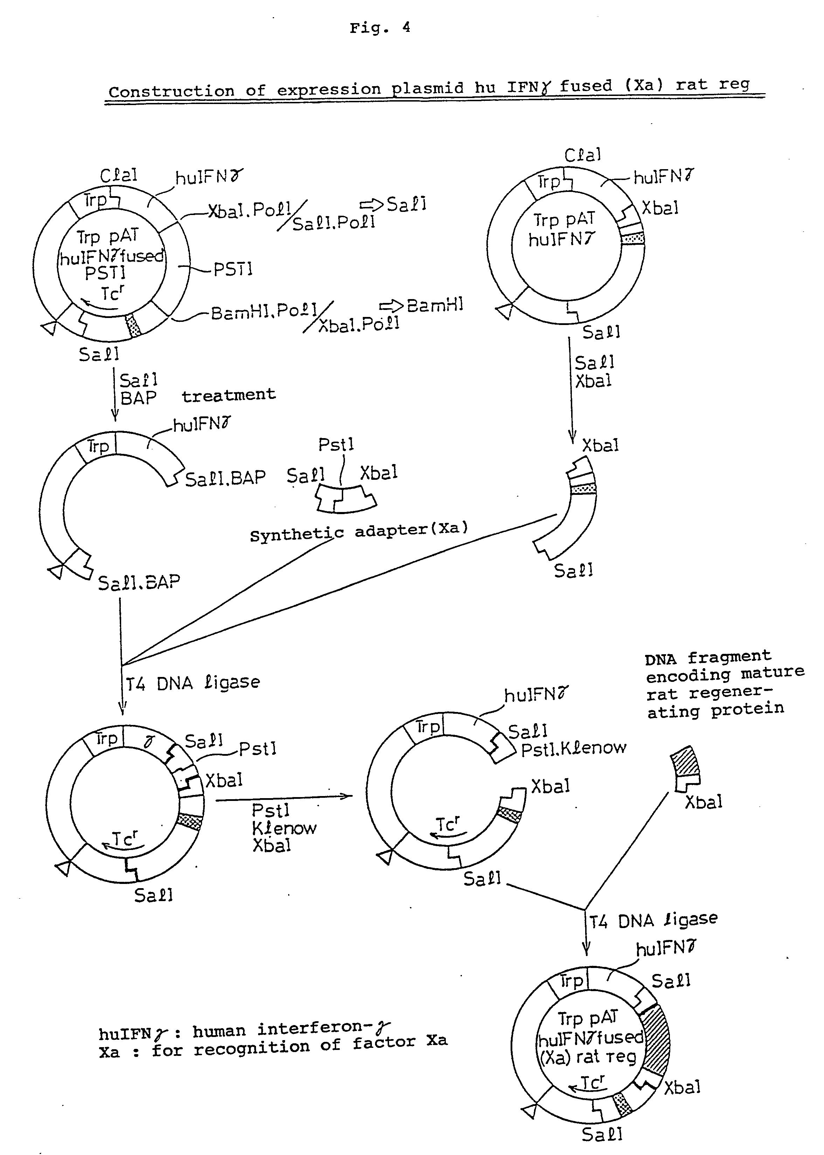

As shown in Figure 4, an expression plasmid (Trp pAT hu-IFNγ fused PSTI, J. Biochem. 102, 607 (1987)) for PSTI fused with human interferon-γ was digested with the restriction enzyme SalI (40 u/μg DNA) at 37°C for 1 hour, and then treated with BAP (0.1 u/pmol) at 37°C for 2 hours. The larger fragment was isolated, and this was then ligated with the oligonucleotide adapter indicated below and with the smaller fragment resulting from prior digestion of the plasmid Trp pAT hu-IFNγ with the restriction enzymes SalI and XbaI.

```
SalI  Asp Ile Glu Gly Arg  PstI  XbaI
 TC GAC ATC GAA GGT CGC TGC AGT
    G TAG CTT CCA GCG ACG TCA GAT C
```

The DNA obtained in this manner was used as the expression vector; this vector possesses, at the adapter site, a DNA sequence encoding the recognition sequence, Ile Glu Gly Arg, for the endopeptidase Factor Xa.

The aforementioned expression vector was digested with the restriction enzyme Pst I (3 u/μg DNA), then treated with Klenow fragment (0.5 u/μg DNA for polymerization, 3 u/μg DNA for digestion) at 37°C for 1 hour, thus forming a Factor Xa recognition site, and was next treated with XbaI at 37°C for 1 hour. Into the vector digested in this manner was inserted a DNA fragment encoding mature rat reg protein obtained by the primer-extension method.

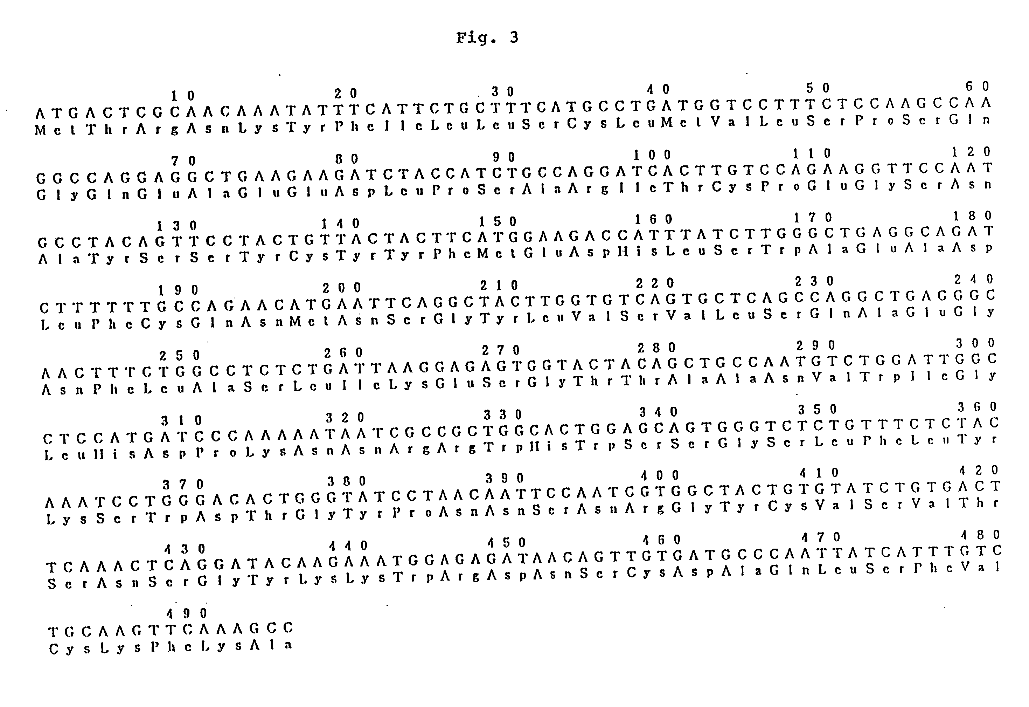

In order to obtain the said DNA fragment encoding mature rat reg protein, a mutation is induced so as to form an XbaI recognition site at a 3´ untranslation site of a single stranded DNA encoding mature rat reg protein and signal peptide (see Fig. 3). Then, a double stranded DNA fragment that encodes mature reg protein without containing signal peptide was prepared by the primer extension method (Nucl. Acids Res. 8, 4057 (1980)), using the following synthetic oligonucleotide.

5´ CAG GAG GCT GAA GAA GAT 3´

The DNA fragment encoding the mature reg protein obtained in this manner was digested with XbaI (3 u/μg DNA) at 37°C for 1 hour, and then inserted into the aforementioned expression vector. The resulting expression vector was designated as Trp pAT hu-IFNγ fused (Xa) rat reg.

An Escherichia coli strain carrying the aforementioned expression vector was designated as Trp pAT

huIFNγ fused (Xa) rat reg/HB101, and deposited with the Fermentation Research Institute Agency for Industrial Science and Technology, 1-3, Higashi 1 chome, Tsukuba-shi, Ibaraki-ken, Japan on November 9, 1988 under Accession Number FERM BP-2142 in accordance with the Budapest Treaty.

(b) Transformation

The Escherichia coli strain C600 was transformed with the aforementioned expression vector. The transformants were cultivated for 16 hours at 37°C on LB plates containing 15 μg/ml of tetracycline, and the transformants containing the target plasmids were selected.

(c) Expression

The aforementioned transformants were cultivated overnight at 37°C in LB medium containing 15 μg/ml of tetracycline, then cultivated at 37°C for 24 hours in a modified M9 medium. In order to analyze the inclusion body, the cells were collected by centrifugation and treated at 4°C for 1 hour with lysozyme (1 mg/ml culture). The cell extract so obtained was ultrasonicated and centrifuged to obtain the inclusion body. This precipitate was suspended in SDS gel sample buffer and concentrated by a factor of 20.

The insoluble fraction (inclusion body) of the protein was analyzed by SDS-PAGE (Nature 227, 680 (1970)), employing Coomassie Brilliant Blue R staining and anti-human interferon-γ antibody.

The 31.9 kilodalton protein obtained in the above-described manner was allowed to react with anti-human interferon-γ antibody and was thereby ascertained to be the target protein.

The band corresponding to the fusion protein of human interferon-γ and reg protein constituted approximately 10% of the total protein content of the E. coli cells.

(d) Isolation and purification

A 3.7 liter batch of the aforementioned recombinant culture was grown for 24 hours and the bacteria were collected by centrifugation. The bacteria were then suspended in buffer A (i.e., 0.1 M Tris-HCl, 5 mM ethylenediamine tetraacetate, 10 mM benzamidine and 0.2 M dithiothreitol, pH 7.5) and distintegrated with a French press or a Gaulin homogenizer. Next, the suspension was subjected to centrifugation to give a pellet and the pellet was washed 3 times with buffer A, thereby obtaining 20.1 g of a pellet. Next, 10 g of the pellet was dissolved in 400 ml of buffer B (i.e., 4 M guanidine hydrochloride, 0.1 M Tris-HCl, 5 mM EDTA, 10 mM BA and 20 mM dithiothreitol, pH 7.5). This solution was diluted to adjust a final concentration of 2 M guanidine hydrochloride and a protein concentration of 1.5 mg/ml, the concentrations of the other constituents being the same as those in buffer B. The protein content was determined with a Bio-Rad Protein Assay Kit. This solution was diluted 5-fold by addition of buffer C (2 M guanidine hydrochloride, concentrations of other constituents the same as in buffer B). This diluted solution was left standing for 1 hour, and the precipitate formed was collected by centrifugation. This precipitate was then washed successively with buffer C and distilled water, and then used for the following enzyme analysis (this specimen will hereinafter be referred to as the fusion protein). The yield of the fusion protein was 10.3 g (wet weight).

A solution containing approximately 3 mg/ml of the fusion protein and 0.055 mg/ml of Lysyl Endopeptidase (final concentrations in each case) was allowed to react for 4 hours at 25°C in 50 mM Tris-HCl (pH 8.0). The reaction mixture was stirred gently, since the protein was undissolved. After completion of the reaction, the precipitated fraction was collected by centrifugation and washed twice with distilled water. This specimen will hereinafter be referred to as specimen A.

Then, 250 mg of specimen A was dissolved in 0.17 ml of a solution containing 6 M urea, 25 mM Tris-HCl and 1 mM dithiothreitol, pH 7.0, and this solution was then subjected to chromatography with Sephadex G-75 (3.2 x 77.0 cm, equilibrated with the above-mentioned buffer solution). A 17 kilodalton protein deduced from the gene was eluted at nearly the void volume. The fraction containing this 17 kilodalton protein was collected and dialyzed against 50 mM Tris-HCl (pH 7.5). Next, the dialysate obtained was centrifuged and the supernatant was collected. The yield was 5.95 mg. A soluble 17 kilodalton protein was contained in this supernatant, and this protein will hereafter be referred to as specimen B.

After dissolving 2 mg of specimen A in 0.5 ml of SDS solution (i.e., 0.06 M Tris-HCl, 2% SDS, 2.5% 2-mercaptoethanol and 10% glycerol), the mixture was treated at 100°C for 5 minutes. Next, the whole amount of the mixture was subjected to SDS-PAGE (13.8 x 15.0 cm, 2 mm, 15% gel, 30 mA, 3 hours electrophoresis). After electrophoresis, the position of the 17 kilodalton protein was ascertained from the staining of the guide strip, and this 17 kilodalton protein was recovered from the remaining gel with a Max-Yield protein recovery unit. The amount recovered was 87 μg. The 17 kilodalton protein was again analyzed by SDS-PAGE, and the results confirmed that the substance was a pure 17 kilodalton protein. The amino acid composition of the 17 kilodalton protein is shown in Table 1. Moreover, investigation of the amino terminus of this protein revealed that the sequence of 14 residues from the amino terminal was Ser-Leu-Val-Asp-Ile-Glu-Gly-Arg-Gln-Glu-Ala-Glu-Glu-Asp. This protein will hereinafter be referred to as specimen C.

The above results demonstrated that the purified 17 kilodalton protein contains the entire sequence of rat reg protein as deduced from the base sequence of the gene, but also had an additional sequence of 8 amino acid residues appended to the amino terminus.

Table 1

| Amino acid composition | | |
| --- | --- | --- |
| Amino acid | Number of amino acid | |
| | Calculated | Found |
| Asp | 20 | 19.8 |
| Glu | 13 | 14.5 |
| Thr | 5 | 5.1 |
| Ser | 21 | 19.0 |
| Pro | 4 | 3.7 |
| Gly | 10 | 14.9 |
| Ala | 11 | 11.0 |
| Cys/2 | 6 | 4.6 |
| Val | 7 | 6.9 |
| Met | 2 | 2.1 |
| Ile | 4 | 4.0 |
| Leu | 12 | 11.8 |
| Tyr | 9 | 8.6 |
| Phe | 6 | 6.1 |
| Lys | 7 | 8.0 |
| His | 3 | 4.1 |
| Trp | 6 | 5.2 |
| Arg | 6 | 5.9 |
| Total | 152 | |

Preparation of monoclonal antibodies

(1) Immunization

The experimental animals used in the preparation of hybridomas and monoclonal antibodies were all BALB/c female mice 8-10 weeks of age.

The proteins used for immunization and used in assays of antibodies were the aforementioned reg protein specimens A, B and C, as well as specimen D that is a reg protein isolated from yeast transformed with the rat reg gene and then purified uniformly.

The first immunization was performed by preparing a 1:1 emulsion of rat reg protein (specimen A) and Freund's complete adjuvant (FCA), and injecting this emulsion subcutaneously into the 8 mice in each of two groups, administering individual doses of 100 μg protein (group 1) or 50 μg protein (group 2).

The second immunization was performed by both subcutaneous and intraperitoneal injections of equal

7

doses of 34 μg (group 1) or 14 μg (group 2) of protein (specimen B) as an emulsion with FCA, at the 41st day after the first immunization.

(2) Determination of serum titer

Blood levels of anti-rat reg protein in the immunized animals were determined by ELISA. First, rat reg protein (specimen B) was aliquoted into wells of a microtiter plate (30 ng protein in 0.1 ml of 0.1 M sodium bicarbonate/well) and left overnight at 4°C to effect coating, after which 0.3 ml aliquots of 1% BSA in phosphate buffered saline (PBS) were added and the plate was incubated at 37°C for 1 hour to effect blocking. Then 0.05 ml per well of the analyte solution was added, and after reaction at 37°C for 1 hour, the assay was performed using a Vectastain ABC Kit (i.e., mouse IgG kit, Vector Laboratories, Inc.) in accordance with the prescribed protocol for the kit. Then, 2 mg/ml of ortho-phenylene diamine (OPD) solution was used as the chromogen, and absorption at 492 nm was measured with MTP-22 Corona microplate photometer.

The titer was measured in the same manner with respect to a guanidine lyzate of Escherichia coli not transformed with the reg protein gene, and two mice with sera displaying comparatively high titers of anti-rat reg protein antibody and low antibody titers to the Escherichia coli guanidine lyzate were used for the preparation of hybridomas.

(3) Preparation of hybridomas

The third immunization was performed 46 days after the second immunization, in the following manner. That is, each mouse was immunized by intraperitoneal injection of a 34 μg dose of reg protein specimen B dissolved in 0.2 ml of PBS. Three days after the third immunization, cell fusion was carried out as follows. The procedure employed for cell fusion was basically the process of Galfre and Milstein [Methods Enzymol. 73, 3 (1981)]. First, mouse myeloma cells (P3/NSI/1-Ag4-1) were cultured in a modified RPMI 1640 medium (i.e., 90% RPMI 1640, 10% fetal calf serum, 0.15 mg/ml sodium pyruvate, 0.15 mg/ml oxaloacetic acid and 0.1 mg/ml kanamycin). Then, 1.37 x $10^7$ cells of these myeloma were mixed with 2.89 x $10^8$ spleen cells from the immunized mice and the cells were pelleted by centrifugation. Next, 1 ml of 48% polyethylene glycol solution was added dropwise to the centrifuge tube containing this pellet over a period of 1 minute, after which the mixture was stirred for 1.5 minutes. Next, 2 ml of serum-free RPMI 1640 medium was added over a period of 2 minutes, then a further 2 ml of the same medium was added over a period of 1 minute, next, another 6 ml of the same medium was slowly added dropwise over a period of 2 minutes while stirring, and finally still another 15 ml of the same medium was gently added. The mixture was centrifuged, thus obtaining a pellet. This pellet was suspended in HAT medium (i.e., 70% RPMI 1640, 10% NCTC 109, 20% fetal calf serum, $10^{-4}$ M hypoxanthine, 4 x $10^{-7}$ M aminopterin, 1.6 x $10^{-5}$ M thymidine, 0.15 mg/ml sodium pyruvate, 0.15 mg/ml oxaloacetic acid, 0.2 IU/ml insulin, 2.5 x $10^{-4}$ M 2-mercaptoethanol, 5 x $10^{-3}$ M HEPES, 0.1 mg/ml kanamycin, and a mixed solution of non-essential amino acids) so as to obtain a spleen cell concentration of 2 x $10^6$ cells/ml. The aforementioned cell suspension was dispensed in aliquots of 0.1 ml per well into 96-well plates which had been separately plated with untreated spleen cells from normal mice (0.95 x $10^5$ cells in 0.1 ml of HAT medium/well), and incubated for 24 hours beforehand. The mixed cells were then cultivated at a temperature of 37°C and humidity 95% or more in an atmosphere of 95% air and 5% carbon dioxide, replacing half of the medium with fresh HAT medium about once every 3 days.

(4) Screening of hybridomas

Approximately 2 weeks later, the production of anti-rat reg protein antibody was examined by testing the supernatant of the proliferated hybridoma cultures. The assay for this purpose was performed by the same procedure as in section 2 above. The supernatant of the hybridomas displaying positive response in this assay was further tested for reactivity with the rat reg protein specimen C as well as the guanidine lyzate of Escherichia coli, and hybridoma lines REG 5, REG 17 and REG 30 producing antibodies reacting specifically with rat reg protein were selected.

(5) Cloning and frozen storage of hybridomas

The aforementioned three lines of hybridoma cells were cloned by the limiting dilution analysis. That is, cells of each line were suspended in RPMI 1640 medium and dispensed into 96-well plates so as to obtain an overall distribution of approximately 0.3 cells in 0.2 ml medium/well. These cells were grown and the titer of the anti-rat reg protein antibody of the medium supernatant was determined in the same manner as in section 4 above. After selection and cultivation of hybridomas producing anti-rat reg protein antibody, the selected hybridomas were suspended in a solution consisting of 90% calf serum and 10% dimethyl sulfoxide. The suspension was frozen and stored.

(6) Preparation of ascites

First, 0.5 ml aliquots of suspensions ($2 \times 10^6$ to $5 \times 10^6$ cells/ml) of the hybridoma cells REG 5, REG 17 AND REG 30 in PBS were injected by the intraperitoneal route into mice which had 7 to 10 days previously received 0.5 ml intraperitoneal doses of Pristane, and about 1 week after the injection of the hybridomas the accumulated ascites fluid was collected by tapping. Then, the ascitic fluid so obtained was centrifuged in Separapid tubes (Sekisui Kagaku, Ltd.) to remove sediment, then aliquoted and placed in frozen storage.

(7) Determination of classes and subclasses of antibodies

The immunoglobulin classes and subclasses of the monoclonal antibodies REG 5, REG 17 and REG 30 were determined by ELISA. The mouse MonoAb•ID•EIA Kit (Zymed Laboratories, Inc.) was used for these identifications. REG 5, REG 17 and REG 30 were classified as IgG 2a($x$), IgM($x$) and IgG 2a($x$), respectively.

(8) Purification of antibodies

REG 5 and REG 30 were purified with an Affigel Protein A MAPS II Kit (BioRad Laboratories, Inc.), in accordance with the prescribed protocol for the kit. REG 17 was purified by gel filtration with Sephadex G-200. The amount of the respective antibodies obtained per milliliter of ascites were 9.4 mg REG 5, 4.1 mg REG 17 and 4.0 mg REG 30.

(9) Preparation of peroxidase conjugates

A conjugate of REG 17 with horseradish peroxidase was prepared in accordance with the method of Nakane et al. (P. K. Nakane and A. Kawaoi, J. Histochem. Cytochem., 22, 1084 (1974)) as follows. First, 0.2 ml of 0.1 M sodium periodate was added to the peroxidase solution (4 mg/ml water), and after reacting for 20 minutes at room temperature, the mixture was dialyzed overnight against 1 mM sodium acetate buffer (pH 4.4) at 4°C, after which the dialysate was adjusted to pH 9.5 with 0.2 M sodium carbonate. Then, REG 17 solution (8 mg protein in 1 ml of 0.01 M sodium bicarbonate) was added, and the mixture was stirred for 2 hours at room temperature. After cooled in an ice bath, the mixture was dialyzed overnight at 4°C against PBS to obtain the desired conjugate.

(10) Sandwich assay of reg protein

A) Using REG 17-peroxidase conjugate

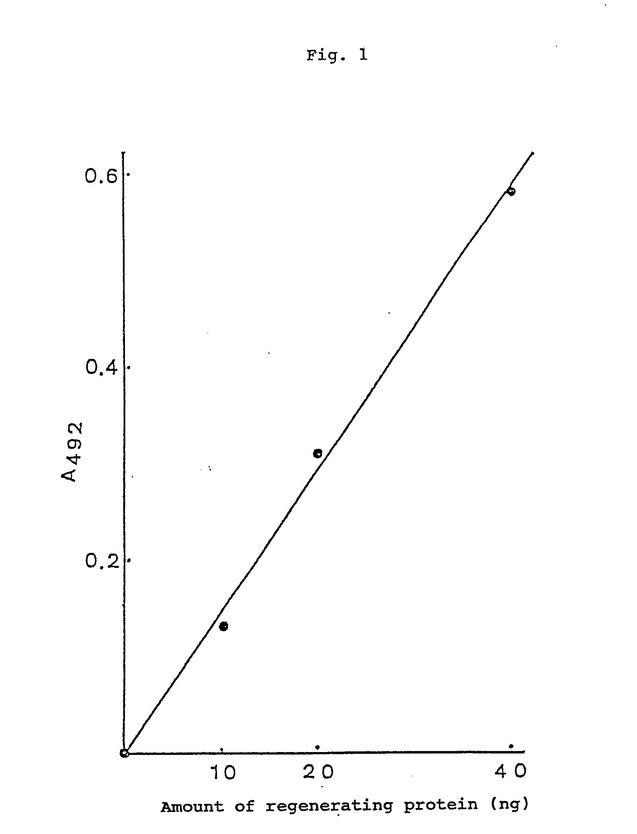

Aliquots of REG 5 solution were dispensed into each well of a microtiter plate (10 μg REG 5 in 0.1 ml of 0.1 mM sodium bicarbonate/well), left standing overnight at 4°C, and after addition of 0.3 ml of 1% casein in PBS, the plate was incubated for 1 hour at 37°C to effect blocking. Then, 0.05 ml aliquots of reg protein (standard or analyte) in 1% BSA/PBS were added and the mixture was allowed to react for 1 hour at 37°C. Then, 0.05 ml aliquots of a 200-fold dilution in 1% BSA/PBS of the REG 17-peroxidase conjugate prepared as in section 9 above were added and the mixture was allowed to react for 30 minutes at room temperature. Next, aliquots of ortho-phenylenediamine (0.5 mg/0.05 ml of 0.1 M citrate buffer, pH 4.2) and hydrogen peroxide (final concentration 0.03%) were added, and again the mixture was allowed to react for minutes at room temperature. Then, the reaction was terminated by addition of 0.1 ml aliquots of 1N sulfuric acid, and the 492 nm absorbance was determined. The detection sensitivity of the reg protein of the present method was 5 ng/well (see Figure 1).

B) Using anti-IgM antibody

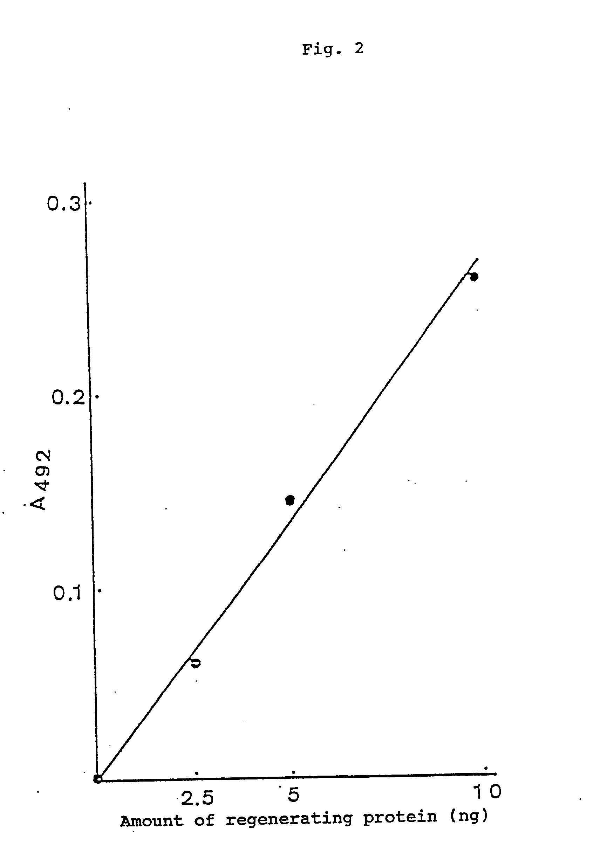

Coating with REG 5, blocking with casein and addition of an analyte containing reg protein were performed as in subsection A above, after which 0.05 ml aliquots of REG 17 solution (20 μg/ml, in 1% BSA/PBS) were added and a reaction was conducted at 37°C for 1 hour. Then, the reagents of a Vectastain mouse IgM kit were added and allowed to react in accordance with the prescribed protocol, thus binding peroxidase to the REG 17 through the anti-IgM antibody. Thereafter, the addition of o-phenylenediamine and the ensuing procedure were carried out as in subsection A above. The detection sensitivity of the reg protein of the present method was 2.5 ng/well (see Figure 2).

(11) Detection of reg protein by the Western blot analysis

A) Detection of recombinant reg protein

Western blotting was carried out with respect to the fusion protein expressed in Escherichia coli as well as to the rat and human reg proteins expressed in yeast. Using the monoclonal antibodies REG 5 and REG 30, coloration was observed at the 32 KD position and at the 16 KD position. These positions agreed with those corresponding to the fusion protein and human and rat reg proteins, respectively. A band that can react with the anti-reg protein antibody was also observed on the low molecular weight side, but the amino acid composition of the protein corresponding to the band and sequencing of the protein verified that this was a partial decomposition product of the reg protein.

B) Detection of rat and human pancreatic reg proteins

When rat and human pancreatic homogenates were subjected to SDS-PAGE, and Western blotting was carried out with respect to the resulting pattern, conspicuous bands at positions corresponding to the reg proteins were visualized by the monoclonal antibodies REG 5 and REG 30. The presence of a protein detected by the aforementioned antibody was also observed on the high molecular side in the case of the human pancreatic analyte, but at the present time the nature of this protein has not yet been clarified.

(12) Sandwich assay of reg protein in normal rat pancreatic and other tissues

Pancreatic and other tissues of normal rats (Lewis strain, female, 5 weeks old) in 0.1 M sodium acetate buffer solution (pH 4.4) containing 1 mM PSMF were crushed with a Polytron respectively, then each lysate obtained was centrifuged at 10,000 x g for 15 minutes and the supernatant was separated. Then, 2% SDS in PBS was added to the sediment, and kept for 1 hour at room temperature. Then, this mixture was again centrifuged for 15 minutes at 10,000 x g, and the supernatant so obtained was regarded as the membrane solubilized fraction. The reg protein content of each of these tissue supernatants and membrane solubilized fractions was measured using the same sandwich assay procedure in section 10 above. An extremely high concentration of reg protein was specifically detected in the supernatant as well as the SDS-solubilized fraction from normal rat pancreas. Using as a reference the coloration of standard recombinant reg protein which was assayed concurrently, the rat pancreas contained approximately 1,080 μg of reg protein per gram wet tissue. Reg protein was also detected in other tissues, but the corresponding amount was small as compared with the pancreas (spleen 36, liver 14, testes 11, brain 10 and kidneys 10 μg per gram wet tissue).

Example 2

Expression of human reg protein in yeast Microorganisms

The host used in the construction of the expression vector was Escherichia coli K-12 (F⁻, hsdR⁺, hsdM⁺, recA⁺, thr, leu, thi, lacY, supE, tonA).

The final host used for the expression was Saccharomyces cerevisiae AH22 (a leu2, his4, can1, cir⁺) (Proc. Natl. Acad. Sci. USA 75, 1929-1933 (1978); Japanese Patent Publication No. 61-55951. The microorganism was deposited with the Fermentation Research Institute Agency of Industrial Science and Technology under Accession Number 312 where it will be maintained under the Budapest Treaty. This yeast was cultivated beforehand in YPDA medium (i.e., 1% yeast extract, 2% polypeptone, 2% glucose and 20 mg/l adenine).

Culture media

Burkholder's minimal medium (Proc. Natl. Acad. Sci. USA 77, 4504-4508 (1980)) was used for the preparation of a Pi-added medium (hereinafter referred to as Pi(+) medium) containing 1.5 g/l of potassium dihydrogen phosphate as well as a Pi-free medium (hereinafter referred to as Pi(-) medium), containing 1.5 g/l of potassium chloride in place of the potassium dihydrogen phosphate.

Enzymes and linkers

Restriction enzymes, T4DNA ligase and Klenow fragment were purchased from Takara Biochemicals, Inc., while XhoI linkers were purchased from Pharmacia.

Plasmids

The yeast-Escherichia coli shuttle vector pAM82 (Proc. Natl. Acad. Sci. USA 80, 1-5 (1983), Japanese Patent Publication No. 61-55951, deposited with the Fermentation Research Institute Agency of Industrial Science and Technology under Accession Number 313, where it will be maintained under the Budapest Treaty was employed as the expression vector. This vector is composed of a 5.5 kb DNA fragment of S. cerevisiae containing ARS1, 2 μm ori and Leu2, and a 3.7 kb pBR322 fragment containing an Apᴿ marker and ori, and also incorporates and 2.7 kb yeast DNA fragment containing the PHO5 promoter.

The pGEM4 plasmids used in the construction of the expression vector were purchased from Promega Biotec (USA).

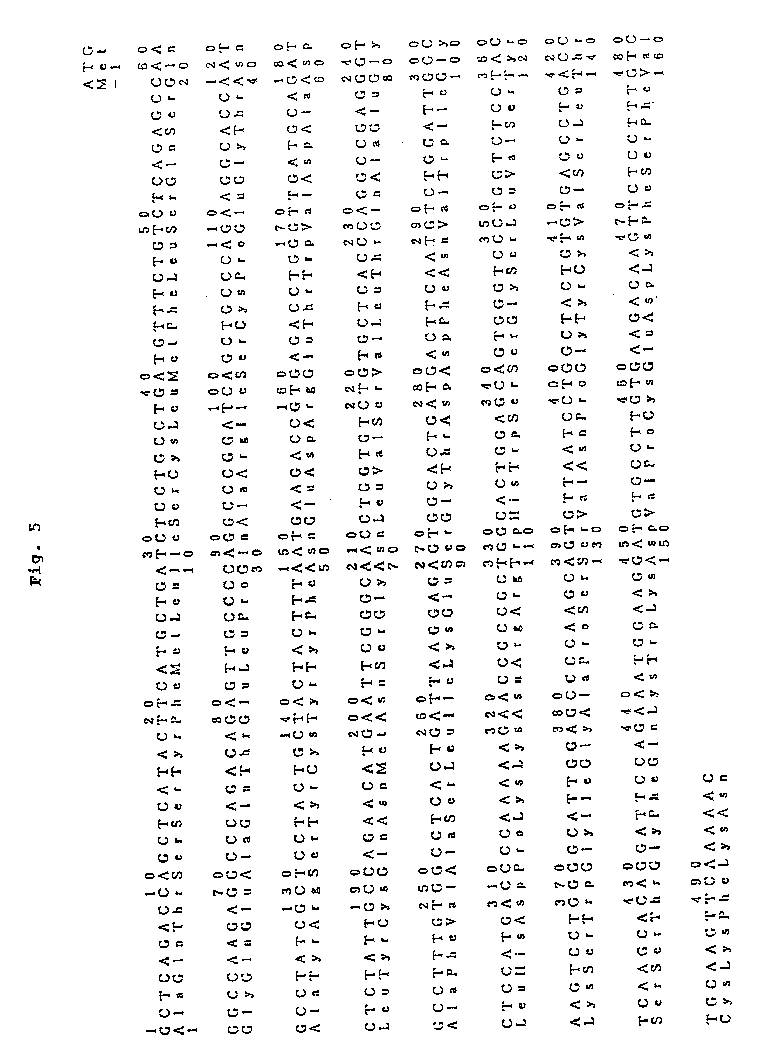

An EcoRI recognition site is present in the structural gene of human reg cDNA (see Figure 5), and therefore this cDNA is divided into two EcoRI fragments when inserted into the EcoRI site of the pBS vector.

Transformation

Transformation was performed as described by Kimura et al (J. Bacteriol. 153, 163-168 (1983)). The Leu⁺ transformants were selected on Burkholder's minimal medium containing 2% agar.

Cultivation and induction

The acid phosphatase promoter (PHO5) can be regulated by the Pi concentration of the culture medium (The EMBO Journal 1, 675-680 (1982)). First, the yeast was aerobically cultivated at 30°C for 2 days in Pi-(+) medium containing histidine (20 μg/ml). Then, 200 μl of this culture was transferred to 10 ml of Pi(-) medium containing 20 μg/ml of histidine. After this had been grown for 3 to 5 days in the same manner, human reg protein was detected in the medium.

11

Construction of expression vector

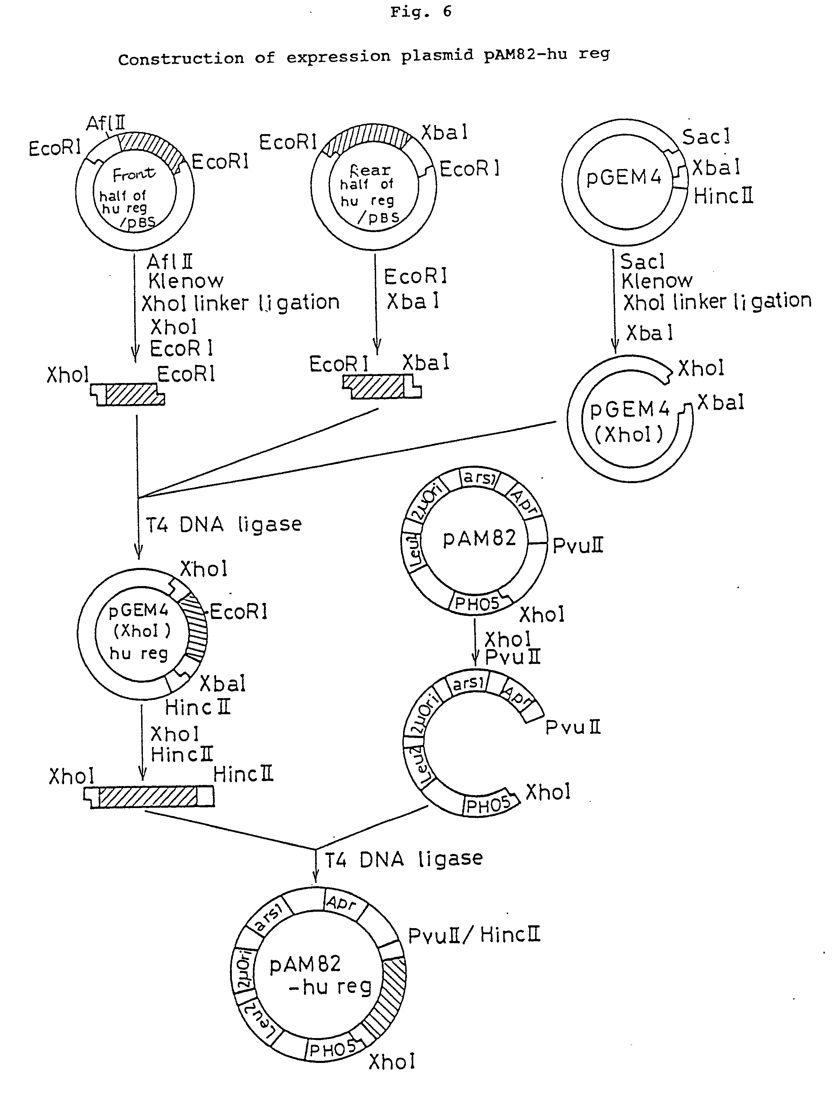

As illustrated in Figure 6, the expression vector was constructed in accordance with conventional methods (Molecular Cloning, Cold Spring Harbor Laboratory, New York (1982)).

The human reg cDNA was inserted into the two pBS vectors in the form of two fragments, i. e., the 5' terminal fragment and the 3' terminal fragment (hereinafter referred to as the front half and rear half, respectively).

First, the pBS vector containing the 5' terminal fragment (front half) was digested with Afl II, treated with Klenow fragment, ligated with a Xhol linker and then digested with EcoRI. The pBS vector containing the 3' terminal fragment (rear half) was digested with EcoRI and Xbal. The plasmid pGEM4 was digested with Sacl, treated with Klenow fragment, ligated with a Xhol linker and then digested with Xbal, resulting in pGEM4 (Xhol). Into the pGEM4 (Xhol), the two human reg cDNA fragments obtained in the manner described above were inserted. The resulting plasmid, designated as pGEM4 (Xhol) human reg, was digested with Xhol and HincII, and the smaller fragment so obtained was inserted into a plasmid pAM82 which had been digested with Xhol and PvuII. The resulting plasmid was designated as pAM82-human reg.

In the above process, all restriction enzymes were used at a concentration of 3 units/μg DNA, and were incubated with the substrates at 37°C for 1 hour.


Production of human reg protein

The expression vector for human reg cDNA constructed in the manner described above was transformed into E. coli K-12 C600, the ampicillin-resistant colonies were selected, and large amount of plasmid DNA was prepared using these recombinant strains.

The recombinant plasmids which had proliferated in E. coli were inserted into the yeast host S. cerevisiae AH22 in accordance with a conventional method and the Leu$^+$ colonies were selected.

The recombinants were grown in a Pi(+) medium for 2 days and then transferred to a Pi(-) medium to induce the production of reg protein. In the above process, the aforementioned yeast was cultivated at 30°C under vigorous agitation in both the Pi(+) and Pi(-) media.

On the 3rd, 4th and 5th days after cultivation in the Pi(-) medium, portions of the culture liquid were sampled and the cells were removed by centrifugation.

The culture liquid so obtained was concentrated by a factor of approximately 1/100 by a Centricon-10 (Amicon) and by passage of nitrogen gas.

Each concentrated culture liquid (corresponding to 0.75 ml of culture liquid) was subjected to 16% SDS-PAGE (FEBS Lett. 58, 254-258 (1975); Nature (London) 227, 680 (1970)), and the resulting patterns were analyzed by staining with Coomassie Brilliant Blue R.

This analysis confirmed the secretory production of human reg protein by the host cells. The detected protein was primarily human reg protein, however, a small amount of contaminant protein was also detected.


Immunological detection of human reg protein

The aforementioned culture liquid was concentrated by a factor of approximately 1/100 by means of a Centricon-10 (Amicon) and the passage of nitrogen gas.

The concentrated culture liquid was subjected to 16% SDS-PAGE, and the resulting patterns were analyzed by Western blotting [J. Virol. 10, 211 (1972)] using the aforementioned monoclonal antibody REG 5 or REG 30, and a 16 kilodalton band was recognized. This reg protein was used in the preparation of monoclonal antibodies.


Purification of human reg protein

The culture liquid containing the reg protein was adjusted to pH 3.4 with 1N hydrochloric acid and diluted with distilled water, thereby adjusting the final electrical conductivity to 0.3 mmho. To this solution was added 100 ml of S-Sepharose (fast flow type) equilibrated with 5 mM sodium acetate buffer (pH 3.4; hereinafter referred to as buffer A), and the mixture was gently stirred for 16 hours at 4°C. This S-Sepharose with adsorbed reg protein was then packed into a column (5 x 8 cm) and washed with buffer A containing 0.3 M sodium chloride, then the protein was eluted with buffer A containing 0.6 M sodium chloride. Then, the reg protein fraction which displayed a molecular weight of approximately 16,000 on the SDS-PAGE (15% gel) pattern was pooled and dialyzed against buffer A for 16 hours at 4°C. This dialysate was then applied to an S-Sepharose column (0.7 x 25 cm) equilibrated with buffer A and the column was then washed with buffer A containing 0.3 M sodium chloride. The adsorbate was eluted under a linear gradient with 0.3-0.6 M sodium chloride solution in an amount equal to 10 times the volume of the column. The reg protein was eluted in buffer A at a sodium chloride concentration of 0.44 M, and displayed a uniform composition on the SDS-PAGE (15% gel) pattern. After desalting by ultrafiltration using an Amicon YM-10 membrane, the purified reg protein was stored at a concentration of 0.1 mg/ml in 10 mM acetic acid. The yield of reg protein was approximately 200 µg per liter of culture liquid.

Characteristics of human reg protein

(a) Molecular weight

The molecular weight of the purified reg protein obtained by the process described above, as determined by SDS-PAGE, was approximately 16,000. This value agrees well with the theoretical value of 16,275 calculated from the amino acid sequence deduced from the cDNA sequence. The amino terminus of the reg protein was inferred as being the 22nd glutamine residue of that corresponding to the cDNA sequence in Fig. 5.

(b) Amino acid composition

The reg protein was desalted by HPLC (Aquapore RP-300 column, Brownlee) and then hydrolyzed for 24 hours at 110°C with 4 M methanesulphonic acid containing 0.2% of 3-(2-aminoethyl) indole. Amino acid analysis was performed with a Hitachi amino acid analyzer Model 835.
As shown in Table 2, the amino acid composition of the reg protein agreed well with the theoretical value.

(c) Amino terminal sequence

When the amino terminal sequence of the reg protein was subjected to analysis using an Applied Biosystems 477A Protein Sequencer, the amino terminus and succeeding amino acids could not be detected at all. Accordingly, the protein was treated with pyroglutamate aminopeptidase and then analyzed in the above manner. The 29 amino acids at the amino terminus of the enzyme-treated reg protein determined in this manner are shown in Table 3. The residue at the amino terminus of this enzyme-treated protein was glutamic acid, and the amino acid sequence (Glu-2 to Glu-30) agreed with the corresponding segment inferred from the cDNA sequence.
Therefore, the results indicated that the amino terminal sequence of the reg protein was the segment extending from the glutamine at the 22nd position or the glutamic acid at the 23rd position of the sequence shown in Figure 5. In rat reg protein, the residue at the amino terminus was glutamic acid, corresponding to the glutamic acid residue at the 23rd position of the amino acid sequence deduced from the cDNA of human reg protein.

Table 2

| Amino acid | Number of amino acid | |
|---|---|---|
| | Calculated | Found |
| Asp | 17.1 | 17 |
| Thr | 6.9 | 7 |
| Ser | 15.7 | 17 |
| Glu | 14.8 | 14 |
| Pro | 6.4 | 6 |
| Gly | 10.2 | 10 |
| Ala | 8.0 | 8 |
| 1/2Cys | 5.5 | 6 |
| Val | 9.9 | 10 |
| Met | 1.0 | 1 |
| Ile | 3.9 | 4 |
| Leu | 8.1 | 8 |
| Tyr | 7.1 | 7 |
| Phe | 7.0 | 7 |
| Lys | 9.1 | 9 |
| His | 2.2 | 2 |
| Arg | 4.8 | 5 |
| Trp | 5.8 | 6 |
| Total | | 144 |

Table 3

| Degradation step | Amino acid residue of reg protein | Recovery (%) |
|---|---|---|
| 1 | (Gln)*1 | |
| 2 | Glu | 17.3 |
| 3 | Ala | 18.1 |
| 4 | Gln | 20.6 |
| 5 | Thr | 11.9 |
| 6 | Glu | 14.9 |
| 7 | Leu | 16.8 |
| 8 | Pro | 15.2 |
| 9 | Gln | 14.7 |
| 10 | Ala | 14.2 |
| 11 | Arg | 7.9 |
| 12 | Ile | 9.8 |
| 13 | Ser | 35.6 |
| 14 | (Cys)*2 | |
| 15 | Pro | 7.5 |
| 16 | Glu | 5.7 |
| 17 | Gly | 8.6 |
| 18 | Thr | 12.2 |
| 19 | Asn | 5.9 |
| 20 | Ala | 6.8 |
| 21 | Tyr | 5.6 |
| 22 | Arg | 3.5 |
| 23 | Ser | 22.0 |
| 24 | Tyr | 4.5 |
| 25 | (Cys)*2 | |
| 26 | Tyr | 4.8 |
| 27 | Tyr | 6.4 |
| 28 | Phe | 4.4 |
| 29 | Asn | 4.1 |
| 30 | Glu | 2.7 |

* 1: Unidentified residue. The residue was inferred to be glutamine from results of treatment with pyroglutamate aminopeptidase.

* 2: Unidentified residue. The residue was inferred to be cysteine on the basis of the cDNA sequence.

## Preparation of monoclonal antibodies

### (1) Immunization

All the experimental animals used for the preparation of hybridomas and the production of monoclonal antibodies were female BALB/c mice 8 weeks of age. The reg proteins used for immunization and assay of antibodies were those prepared by the procedures described above.

First immunization: A solution of human reg protein in PBS was prepared as an emulsion with Freund's complete adjuvant (FCA) in the ratio 1:1, and injected subcutaneously into 21 mice at the dosage of 20 μg protein per mouse.

Second immunization: This was performed 32 days after the first immunization. Human reg protein as an emulsion with FCA was injected subcutaneously at the dosage of 5 μg (group 1, 10 mice) or 20 μg (group 2, 11 mice).

Third and fourth immunizations: These were performed 55 days and 69 days, respectively, after the second immunization, and by the same method as the second immunization.

(2) Determination of serum titer

The blood levels of anti-human reg protein in the immunized animals were measured by ELISA. More specifically, aliquots of human reg protein were dispensed into a microtiter plate (30 ng protein in 0.1 ml of 0.1 M sodium bicarbonate/well), and the plate was left overnight at 4°C to effect coating, after which 0.3 ml aliquots of 1% BSA/PBS were added to each well and the plate was incubated for 1 hour at 37°C to effect blocking. Then, 0.05 ml aliquots of the analyte solution were added to each well, and after reaction at 37°C for 1 hour the assay was performed with a Vectastain ABC Kit (mouse IgG kit, Vector Laboratories, Inc.) in accordance with the prescribed protocol for the kit. A 1 mg/ml solution of o-phenylenediamine was used as the chromogen, and the 492 nm absorbance was measured with an MTP-22 corona microplate photometer. The mice with sera displaying a high titer of anti-human reg protein antibody were used for the preparation of hybridomas.

(3) Preparation of hybridomas

Experiment 1

A fifth immunization was performed 33 days after the fourth immunization as follows. More specifically, 45 g of human reg protein was dissolved in 0.2 ml of phosphate buffered saline and injected intraperitoneally. Then, 3 days after the fifth immunization, cell fusion was performed as follows. The fusion was performed substantially in accordance with the method of Galfre and Milstein (Methods Enzymol. 73, 46 (1981)). First, mouse myeloma cells (P3-NS1-1-Ag4-1) were cultured in a modified RPMI 1640 medium (i.e., 90% RPMI 1640, 10% fetal calf serum, 0.15 mg/ml sodium pyruvate, 0.15 mg/ml oxaloacetic acid and 0.1 mg/ml kanamycin), then $3.93 \times 10^7$ of these cells were combined with $1.34 \times 10^8$ spleen cells from the immunized mice and centrifuged to form a pellet in a centrifuge tube. Then, 1 ml of 48% polyethylene glycol 4000 was added dropwise, over a period of 1 minute, followed by agitation for 1.5 minutes. Then 2 ml of serum-free RPMI 1640 medium was added over a period of 2 minutes, then a further 2 ml of the same medium was added over a period of 1 minute, next, 6 ml of the same medium was slowly added dropwise over a period of 2 minutes while stirring, and finally 12 ml of the same medium was gently added and then pelleted by centrifugation. This pellet was suspended in HAT medium (i.e., 70% RPMI 1640, 10% NCTC 109, 20% fetal calf serum, $10^{-4}$ M hypoxanthine, $4 \times 10^{-7}$ M aminopterin, $1.6 \times 10^{-5}$ M thymidine, 0.15 mg/ml sodium pyruvate, 0.15 mg/ml oxaloacetic acid, 0.2 IU/ml insulin, $2.5 \times 10^{-4}$ M 2-mercaptoethanol, $5 \times 10^{-3}$ M HEPES, 0.1 mg/ml kanamycin and a mixed solution of nonessential amino acids), the spleen cell concentration being adjusted to $1.5 \times 10^6$ cells/ml. Then, 0.1 ml aliquots of this cell suspension were dispensed into the wells of a 96-well plate and the cells were cultured in a atmosphere of 95% air and 5% carbon dioxide at a temperature of 37°C and humidity of 95% or more. After 3 days of growth, a further 0.1 ml of HAT medium was added to each well, and subsequently half of the medium was replaced by fresh HAT medium as needed.

Experiment 2

A fifth immunization was performed 46 days after the fourth, in the same manner as in Experiment 1, and cell fusion was also performed 3 days after the fifth immunization. The mouse myeloma cell line used was P3X63-Ag 8.653 ($6.27 \times 10^7$ cells), and these myeloma cells were fused with $2.65 \times 10^8$ mouse spleen cells by the same method as in Experiment 1. After fusion treatment, the cells were suspended in HAT medium at a concentration of $1.3 \times 10^6$ cells/ml, and this suspension was dispensed into 96-well plates in aliquots of 0.1 ml per well. The procedures for addition and replacement of HAT medium as well as other culture conditions, etc., were the same as in Experiment 1.

(4) Screening of hybridomas

16

After approximately 2 weeks of growth, the production of human reg protein was examined by analyzing the supernatant of the culture medium. The assay was conducted by the same method as in section 2 above. Hybridomas which displayed stable production of antibodies reacting specifically with human reg protein were selected.

(5) Cloning and frozen storage of hybridomas

The aforementioned hybridoma cells were cloned by the limiting dilution analysis. More specifically, the cells were suspended in RPMI 1640 medium and dispensed into 96-well plates so as to achieve a distribution of 0.3 cells in 0.2 ml medium/well. The titer of the antibody of the anti-human reg protein in the supernatant of the medium was determined by the same procedure as in section 4 above, leading to the selection of the hybridoma clones HREG N1 and HREG 9 which produce anti-human reg protein antibodies, respectively. After suitable periods of growth, these hybridomas were frozen and stored in a freezing solution consisting of 90% calf serum and 10% dimethyl sulfoxide.

(6) Preparation of ascites

First, 0.5 ml doses of a PBS suspension of hybridoma cells ($2 \times 10^6$ to $5 \times 10^6$ cells/ml) were administered intraperitoneally to mice which had been primed 7-10 days beforehand by intraperitoneal injection of 0.5 ml Pristane, and after approximately 1 week, the accumulated ascites fluid was collected by tapping. The sediment was removed from the ascites fluid by centrifugation in Separapid tubes (Sekisui Kagaku), after which the fluid was aliquoted and placed in frozen storage. The amount of antibody obtained per ml of ascites fluid was 1.9 mg and 4.4 mg for HREG N1 and HREG 9, respectively.

(7) Determination of antibody classes and subclasses

The immunoglobulin classes and subclasses of the monoclonal antibodies produced by the hybridoma cells were determined by ELISA, using MonoAb ID EIA Kit (Zymed Company). The results showed that HREG N1 was classified as IgG2a ($x$) and that HREG 9 was classified IgM ($x$)

(8) Purification of antibodies

The monoclonal antibody HREG N1 was purified using an Affigel Protein A MAPS II Kit (BioRad Laboratories, Inc.), in accordance with the prescribed protocol for the kit. The monoclonal antibody HREG 9 was purified by ammonium sulfate fractionation (50% saturation).

(9) Sandwich assay of human reg protein

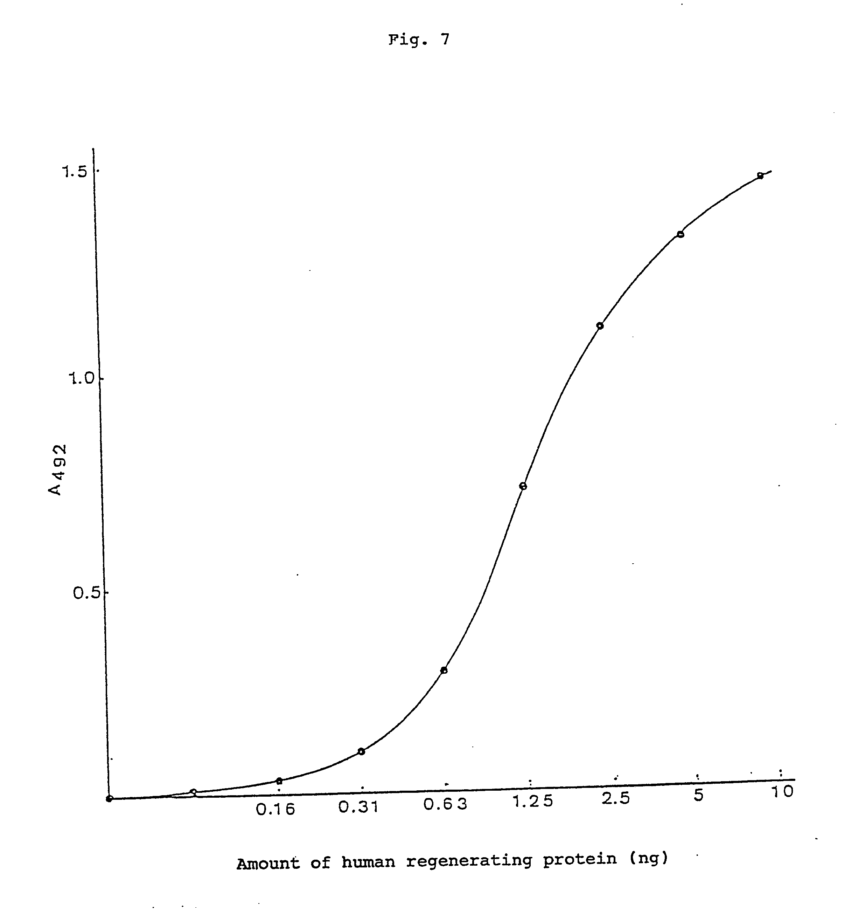

An HREG N1 solution was aliquoted into a microtiter plate (0.5 $\mu$g/0.1 ml of 0.1 M sodium carbonate/well), and after the plate was allowed to stand overnight at 4°C, 0.3 ml aliquots of 1% BSA (in phosphate buffered saline) were added and the plate was incubated for 1 hour at 37°C to effect blocking. Then, 0.05 ml aliquots of human reg protein solution (in 1% BSA/PBS) were added and the mixture was allowed to react for 1 hour at 37°C. Next, a 0.05 ml aliquot of a 4,000-fold dilution of a peroxidase conjugate of the monoclonal antibody HREG 9 (prepared in the same manner as in Example 1) was added to each well, and after a 2 hour reaction at 37°C, aliquots of o-phenylenediamine solution (0.01 mg/0.1 ml of 0.1 M citrate buffer solution, pH 4.2) and hydrogen peroxide (final concentration 0.03%) were added, and the mixture was allowed to react for 20 minutes at room temperature. Then, the reaction was terminated by addition of 0.1ml aliquots of IN sulfuric acid, and the absorption was measured at 492 nm. The detection sensitivity of the present method with respect to human reg protein was 0.16 ng/well (see Figure 7).

**Claims**

1. A monoclonal antibody recognizing a reg protein.

2. A monoclonal antibody according to claim 1, wherein said reg protein is of human or rat origin.

3. A monoclonal antibody according to claim 1, wherein said monoclonal antibody is selected from the group consisting of monoclonal antibody REG 5, monoclonal antibody REG 17, monoclonal antibody REG 30, monoclonal antibody HREG N1 and monoclonal antibody HREG 9.

4. A monoclonal antibody according to any one of claims 1 to 3, wherein said monoclonal antibody is immobilized upon a solid support.

5. A monoclonal antibody according to any one of claims 1 to 3, wherein said monoclonal antibody is enzyme-labelled.

6. A hybridoma producing a monoclonal antibody of any one of claims 1 to 3.

7. A hybridoma according to claim 6, wherein said hybridoma is one selected from the group consisting of hybridoma REG 5, hybridoma REG 17, hybridoma REG 30, hybridoma HREG N1 and hybridoma HREG 9.

8. A method for the assay of a reg protein, comprising the steps of,
preparing a first antibody that specifically recognizes a reg protein,
adding an analyte containing said reg protein so as to bind said reg protein to said first antibody,
adding a second antibody that specifically recognizes said reg protein so as to bind said second antibody to said conjugated reg protein,
adding a third antibody that specifically recognizes said second antibody so as to bind said third antibody to said conjugated second antibody, and
determining the amount of said reg protein by measuring the amount of said third antibody.

9. A method according to claim 8, wherein said second antibody is an IgM and said third antibody is an anti-IgM antibody.

10. A method for the assay of reg protein, comprising the steps of,
preparing a first antibody that specifically recognizes a reg protein,
adding an analyte containing said reg protein so as to bind said reg protein to said first antibody,
adding a labelled second antibody that specifically recognizes said reg protein so as to bind said second antibody to said conjugated reg protein, and
determining the amount of said reg protein by measuring the amount of said labelled second antibody.

11. A method according to claim 10, wherein said second antibody is enzyme-labelled.

12. A method according to claim 11, wherein said enzyme is a peroxidase.

18

Fig. 1

Amount of regenerating protein (ng)

Fig. 2

Fig. 3

```
          10                20                30                40                50                60
ATGACTCGCAACAAATATTTCATTCTGCTTTCATGCCTGATGGTCCTTTCTCCAAGCCAA
Met Thr Arg Asn Lys Tyr Phe Ile Leu Leu Ser Cys Leu Met Val Leu Ser Pro Ser Gln

          70                80                90               100               110               120
GGCCAGGAGGCTGAAGAAGATCTACCATCTGCCAGGATCACTTGTCCAGAAGGTTCCAAT
Gly Gln Glu Ala Glu Glu Asp Leu Pro Ser Ala Arg Ile Thr Cys Pro Glu Gly Ser Asn

         130               140               150               160               170               180
GCCTACAGTTCCTACTGTTACTACTTCATGGAAGACCATTTATCTTGGGCTGAGGCAGAT
Ala Tyr Ser Ser Tyr Cys Tyr Tyr Phe Met Glu Asp His Leu Ser Trp Ala Glu Ala Asp

         190               200               210               220               230               240
CTTTTTTGCCAGAACATGAATTCAGGCTACTTGGTGTCAGTGCTCAGCCAGGCTGAGGGC
Leu Phe Cys Gln Asn Met Asn Ser Gly Tyr Leu Val Ser Val Leu Ser Gln Ala Glu Gly

         250               260               270               280               290               300
AACTTTCTGGCCTCTCTGATTAAGGAGAGTGGTACTACAGCTGCCAATGTCTGGATTGGC
Asn Phe Leu Ala Ser Leu Ile Lys Glu Ser Gly Thr Thr Ala Ala Asn Val Trp Ile Gly

         310               320               330               340               350               360
CTCCATGATCCCAAAAATAATCGCCGCTGGCACTGGAGCAGTGGGTCTCTGTTTCTCTAC
Leu His Asp Pro Lys Asn Asn Arg Arg Trp His Trp Ser Ser Gly Ser Leu Phe Leu Tyr

         370               380               390               400               410               420
AAATCCTGGGACACTGGGTATCCTAACAATTCCAATCGTGGCTACTGTGTATCTGTGACT
Lys Ser Trp Asp Thr Gly Tyr Pro Asn Asn Ser Asn Arg Gly Tyr Cys Val Ser Val Thr

         430               440               450               460               470               480
TCAAACTCAGGATACAAGAAATGGAGAGATAACAGTTGTGATGCCCAATTATCATTTGTC
Ser Asn Ser Gly Tyr Lys Lys Trp Arg Asp Asn Ser Cys Asp Ala Gln Leu Ser Phe Val

         490
TGCAAGTTCAAAGCC
Cys Lys Phe Lys Ala
```

EP 0 369 797 A2

## Fig. 4

Construction of expression plasmid hu IFNγ fused (Xa) rat reg

huIFNγ : human interferon-γ
Xa : for recognition of factor Xa

Fig. 5

EP 0 369 797 A2

```
                                                                                    A T G
                                                                                    M e t
                                                                                    - 1

 1                 1 0                 2 0                 3 0                 4 0                 5 0                 6 0
G C T C A G A C C A G C T C A T A C T T C A T G C T G A T C T C C T G C C T G A T G T T T C T G T C T C A G A G C C A A
A l a G l n T h r S e r S e r T y r P h e M e t L e u I l e S e r C y s L e u M e t P h e L e u S e r G l n S e r G l n
 1                                                                       1 0                                         2 0

                  7 0                 8 0                 9 0                1 0 0                1 1 0                1 2 0
G G C C A A G A G G C C C A G A C A G A G T T G C C C C A G G C C C G G A T C A G C T G C C C A G A A G G C A C C A A T
G l y G l n G l u A l a G l n T h r G l u L e u P r o G l n A l a A r g I l e S e r C y s P r o G l u G l y T h r A s n
                                                   3 0                                                               4 0

                 1 3 0                1 4 0                1 5 0                1 6 0                1 7 0                1 8 0
G C C T A T C G C T C C T A C T G C T A C T A C T T T A A T G A A G A C C G T G A G A C C T G G G T T G A T G C A G A T
A l a T y r A r g S e r T y r C y s T y r T y r P h e A s n G l u A s p A r g G l u T h r T r p V a l A s p A l a A s p
                                                   5 0                                                               6 0

                 1 9 0                2 0 0                2 1 0                2 2 0                2 3 0                2 4 0
C T C T A T T G C C A G A A C A T G A A T T C G G G C A A C C T G G T G T C T G T G C T C A C C C A G G C C G A G G G T
L e u T y r C y s G l n A s n M e t A s n S e r G l y A s n L e u V a l S e r V a l L e u T h r G l n A l a G l u G l y
                                                   7 0                                                               8 0

                 2 5 0                2 6 0                2 7 0                2 8 0                2 9 0                3 0 0
G C C T T T G T G G C C T C A C T G A T T A A G G A G A G T G G C A C T G A T G A C T T C A A T G T C T G G A T T G G C
A l a P h e V a l A l a S e r L e u I l e L y s G l u S e r G l y T h r A s p A s p P h e A s n V a l T r p I l e G l y
                                                   9 0                                                              1 0 0

                 3 1 0                3 2 0                3 3 0                3 4 0                3 5 0                3 6 0
C T C C A T G A C C C C A A A A A G A A C C G C C G C T G G C A C T G G A G C A G T G G G T C C C T G G T C T C C T A C
L e u H i s A s p P r o L y s L y s A s n A r g A r g T r p H i s T r p S e r S e r G l y S e r L e u V a l S e r T y r
                                                  1 1 0                                                             1 2 0

                 3 7 0                3 8 0                3 9 0                4 0 0                4 1 0                4 2 0
A A G T C C T G G G G G C A T T G G A G C C C C A A G C A G T G T T A A T C C T G G C T A C T G T G T G A G C C T G A C C
L y s S e r T r p G l y I l e G l y A l a P r o S e r S e r V a l A s n P r o G l y T y r C y s V a l S e r L e u T h r
                                                  1 3 0                                                             1 4 0

                 4 3 0                4 4 0                4 5 0                4 6 0                4 7 0                4 8 0
T C A A G C A C A G G A T T C C A G A A A T G G A A G G A T G T G C C T T G T G A A G A C A A G T T C T C C T T T G T C
S e r S e r T h r G l y P h e G l n L y s T r p L y s A s p V a l P r o C y s G l u A s p L y s P h e S e r P h e V a l
                                                  1 5 0                                                             1 6 0

                 4 9 0
T G C A A G T T C A A A A A C
C y s L y s P h e L y s A s n
```

Fig. 6

Construction of expression plasmid pAM82-hu reg

Fig. 7

Amount of human regenerating protein (ng)